# EUROPEAN PATENT APPLICATION

(11) **EP 3 998 087 A2**
(43) Date of publication of application: **18.05.2022**
(21) Application number: 21204399.6
(22) Date of filing: 25.10.2021
(51) Int. Cl.: A61L 9/14, A61L 2/22, B05B 1/14

(54) **EQUIPMENT FOR NEBULISING OR ATOMISING A SANITIZING AND STERILIZING SUBSTANCE**

(30) Priority: 28.10.2020 IT 202000006031 U
(71) Applicant: Wow Kemical S.r.l., 35010 Vigonza (PD) (IT); Smart Fluids S.r.l., 46100 Mantova (IT)
(72) Inventor: MARIN, Adriano, 35030 Baone (PD) (IT); BROGNOLI, Matteo, 46040 Cavriana (MN) (IT); SOMENSARI, Nicola, 46100 Mantova (IT)
(74) Representative: De Ros, Alberto

(57) **Abstract**

Equipment (1000, 2000) for nebulising or atomising a sanitizing and sterilising substance (S) in an environment, said substance (S) comprising hydrogen peroxide, at least one organic acid, at least one solvent, at least one surfactant and water.

The equipment (1000) comprises at least one nozzle (100) having at least one inlet and at least one outlet and being adapted to eject the sanitizing and sterilizing substance (S).

The nozzle (100) is fluidically connected to at least one duct (210) adapted to be supplied with the sanitizing and sterilizing substance (S), preferably under pressure, and is adapted to generate a jet (110) comprising the sanitizing and sterilizing substance (S).

## Description

### FIELD OF THE INVENTION

The present invention relates to an equipment for nebulising or atomising a sanitizing and sterilizing substance into the air of an environment.

### BACKGROUND ART

The recent Sars Covid-19 pandemic has necessarily increased attention on sanitizing and disinfecting procedures intended both for people and for environments. These latter in particular must be protected against potential contamination resulting from crowding thereof, such as hospitals, transport vehicles, tunnels or sterilisation environments for people and vehicles, hotels, restaurants, commercial outlets, cinemas, theatres and homes.

They are known sanitizing and/or sterilizing solutions by dry or wet nebulisation processes of commercially available sanitizing substances, but these are often harmful to aquatic organisms and/or toxic and/or alcoholic and/or flammable. In other words, the risk for humans, animals or the environment resulting from exposure to these substances is not negligible.

Typically, such processes must be performed without people and/or animals within the environment to be treated and require long delivery and contact times of the substance, such as 40-60 minutes.

### SUMMARY

The general object of the present invention is to overcome the drawbacks of the prior art, in particular to provide an equipment for nebulising or atomising a sanitizing and sterilizing substance into an environment, in particular a non-toxic and environmentally friendly sanitizing and sterilizing substance, capable of keeping such an environment liveable during the nebulisation or atomisation process.

A further specific object of the present invention is to provide an equipment for nebulising or atomising a sanitizing and sterilizing substance into an environment without causing condensation on the walls with short substance delivery and contact times.

This general object as well as other further specific objects are reached thanks to what set forth in the appended claims that form an integral part of the present description.

### LIST OF FIGURES

The present invention shall become more readily apparent from the detailed description that follows to be considered together with the accompanying drawings in which:
Fig. 1 shows very schematically an example of a first embodiment of an equipment according to the present invention,
Fig. 2 shows schematically an example of a second embodiment of an equipment according to the present invention.

As it can be easily understood, there are various ways of practically implementing the present invention which is defined in its main advantageous aspects in the appended claims and is not limited either to the following detailed description or to the appended drawings.

### DETAILED DESCRIPTION

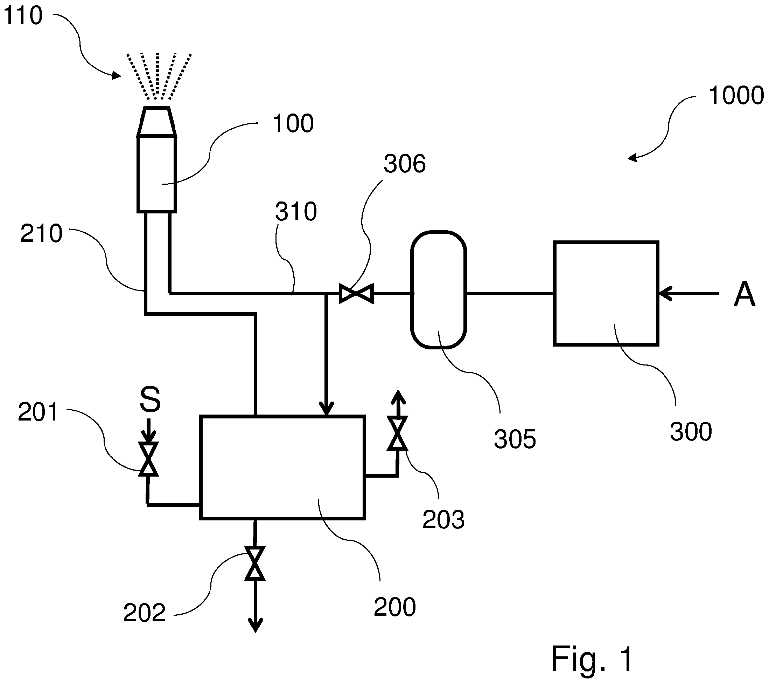

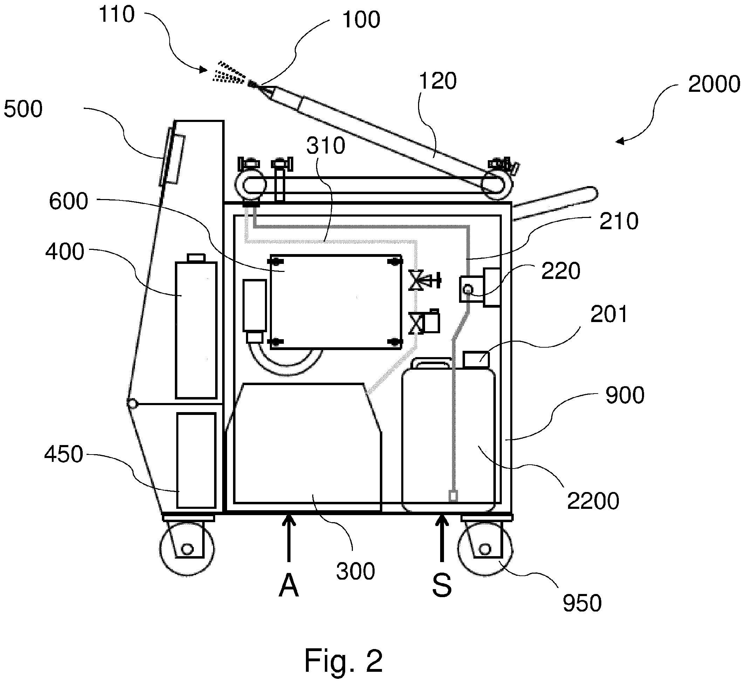

In Fig. 1 it is shown, schematically and in a simplified manner, a system of an equipment for nebulising or atomising a sanitizing and sterilizing substance S in an environment according to the present invention, referred to as a whole by the numerical reference 1000 in Fig. 1 and 2000 in Fig. 2.

It will be hereinafter referred to by the abbreviated notation "equipment 1000" or "equipment 2000".

According to an alternative use of the present invention, the equipment 1000 or 2000 may be further used to nebulise or atomise a sanitizing and sterilizing substance S on a surface, in particular on the surface of sewage or sludge, for example to treat infected sewage, or on plant surfaces, for example to treat plant pathologies.

The equipment 1000 is configured to form a fixed system, e.g. integrated in a building or on a means of transport. The equipment 2000 is configured to form a movable system, with the possibility of being moved to different environments as required.

The equipment 1000 or 2000 comprises at least one nozzle 100, having at least one input and at least one output, in particular at least two inputs.

Advantageously, at least one inlet of the nozzle 100 is fluidically connected to at least one duct 210 adapted to be supplied with the sanitizing and sterilizing substance S.

In particular, an outlet of the nozzle 100 is adapted to generate a jet 110 comprising the sanitising and sterilising substance S.

With reference to Fig. 1, the duct 210 is connected at a first end to the nozzle 100, in particular to an inlet of the nozzle 100, and at a second end to a tank 200 adapted to contain the sanitizing and sterilizing substance S.

According to a first preferred embodiment, the equipment 1000 is configured to form a fixed system.

Advantageously, the equipment 1000 comprises a plurality of nozzles 100 having at least one inlet and at least one outlet and being capable of ejecting the sanitizing and sterilizing substance S, and a hydraulic network adapted to distribute the sanitizing and sterilizing substance S to the plurality of nozzles 100.

Preferably, the sanitizing and sterilizing substance S is under pressure.

In particular, the tank 200 is kept under pressure, i.e. the fluid contained within the tank 200 has a pressure different from the ambient pressure, typically greater than the ambient pressure. Such a pressure is adapted to ensure that the sanitising and sterilising substance S is introduced into the duct 210 and subsequently ejected from the nozzle 100. For example, the tank can keep the fluid inside thereof at a pressure of 4-5 bars.

The tank 200 (in Fig. 1) or 2200 (in Fig. 2) comprises, for example, an opening 201 adapted to allow the sanitising and sterilising substance S enter the tank.

In particular, the opening 201 comprises a closure device, preferably an air-tight cap, adapted to be removed by a user to allow the sanitising and sterilising substance S enter the tank.

Typically, the tank 200 (in Fig. 1) or 2200 (in Fig. 2) comprises at least one level sensor; such sensor measures the free surface quota of the sanitizing and sterilizing substance S contained within the tank. Such measurement can be carried out directly, e.g. by means of an electrical or electronic device which is sensitive to contact with a liquid, or by an electrical or electronic device of the optical type. Alternatively, such a measurement may be carried out indirectly by, for example, measuring (typically continuously or repeatedly or periodically) the weight of the tank and the liquid therein; for example, as we know that the tank when considered as "empty" weighs A Kg and when considered as "full" weighs B Kg, the liquid level (in %) or filling level L (in %) if, at a certain instant, M Kg is measured, is given by the formula L = (M-A)/(B-A)*100.

When the sensor detects that the free surface of the sanitising and sterilising substance S is below a minimum level, the sensor may signal to the user (e.g. via an electronic unit electrically connected to the sensor) that an additional amount of sanitizing and sterilizing substance S must be introduced into the tank to bring the free surface above the minimum quota, so that the correct operation of the equipment 1000 or 2000 is guaranteed, or that it is necessary to replace the container if it is of an interchangeable type.

Advantageously, when the level sensor detects that the free surface of the sanitizing and sterilizing substance S has reached a maximum threshold, the sensor signals the user to stop introducing the sanitizing and sterilizing substance S into the tank.

Advantageously, the tank 200 (in Fig. 1) or 2200 (in Fig. 2) further comprises at least one valve, preferably two valves, for example a first safety valve 202 and a second vent valve 203, adapted to prevent over-pressures inside the tank.

Typically, the geometric dimensions of the tank vary depending on the type of system (fixed or movable) and/or depending on the environment in which the substance S is to be nebulised or atomised.

Advantageously, the tank 200 (in Fig. 1) or 2200 (in Fig. 2) may be sized to secure a "daily charge", i.e. secure a set number of sprays with a given amount of sanitising and sterilising substance S contained in the tank, while keeping the free surface of the substance above the minimum threshold.

According to a second preferred embodiment, the equipment 2000 is configured to constitute a movable system.

With reference to Fig. 2, the equipment 2000 is comprised in a movable casing, for example a crate 900 provided with at least two wheels 950, preferably four wheels 950.

Typically, the tank 2200 of this embodiment is not a tank kept under pressure, i.e. the fluid contained within the tank 2200 has a pressure substantially equal to the ambient pressure. Typically, the sanitizing and sterilizing substance S is introduced into the duct 210 by means of a pump 220 (see Fig. 2).

Advantageously, downstream of the tank 200 (in Fig. 1) or 2200 (in Fig. 2) a metering device, such as a gear metering device, regulating the amount of sanitizing and sterilizing substance to be supplied to the nozzle 100, i.e. the duct 210, is provided.

Advantageously, the equipment 1000 or 2000 further comprises at least one compressor 300 fluidically connected at inlet to an environment and at outlet to the nozzle 100.

In particular, the compressor 300 draws in air A from the environment and compresses it to a pressure greater than the ambient pressure. For example, the compressor 300 compresses air A to a pressure of 8 bars.

Advantageously, the compressed air A leaving the compressor 300 is cooled and/or dehumidified, for example by means of a forced ventilation system.

According to a first preferred embodiment, the compressed air A is subsequently stored in a specific receptacle 305, such as a sealed tank, placed along at least one duct 310.

Advantageously, the receptacle 305 is fluidically connected to the tank 200.

In particular, the receptacle 305 supplies compressed air A to the tank 200 in order to keep the tank 200 under pressure, in particular to keep the fluid inside the tank 200 under pressure.

Typically, the compressed air A exiting the receptacle 305 and entering the tank 200 undergoes a pressure reduction, for example by means of a pressure regulator 306 placed downstream of the receptacle 305.

For example, the pressure of the compressed air A entering the tank 200 may be reduced so that it is substantially equal to the internal pressure of the tank 200 which ensures that the sanitising and sterilising substance S is introduced into the duct 210.

According to a first preferred embodiment and with reference to Fig.1, the compressor 300 is actuated by a motor (not shown in the figure) connected to a power supply source, typically the power grid.

According to a second preferred embodiment and with reference to Fig.2, the compressor 300 is actuated by a motor (not shown in the figure) connected to a battery 400, for example a 24 V battery.

Advantageously, the equipment 2000 further comprises a battery charger 450, typically comprising a plug adapted to be connected to the power grid, a circuit breaker and a transformer.

Advantageously, the equipment 1000 or 2000 further comprises an electronic unit 600 (typically computerised and programmable). For example, the electronic unit 600 can control: the operation of the sensors and valves of the equipment 1000 or 2000, in particular the level sensor of the tank 200 (in Fig. 1) or 2200 (in Fig. 2), the charge level of the battery 400, the connection status of the battery charger 450 to the power grid, the dispenser 201.

In particular, the electronic unit 600 is connected to a human-machine interface 500.

Advantageously, the human-machine interface 500 comprises a display and/or buttons and/or a speaker and/or a touch screen.

With reference to Fig. 2, the ends of the duct 210 and the duct 310 connected to the nozzle constitute an extensible portion 120 of the equipment 2000.

For example, said extensible portion 120 may be an extensible arm or a flexible deformable duct capable of being moved by a user, wherein at the distal end of the extensible portion 120 the nozzle 100 is located.

Advantageously, the duct 210 is connected to the nozzle 100 via a first inlet of the nozzle and the duct 310 is connected to the nozzle 100 via a second inlet of the nozzle; in other words, the supply of the sanitizing and sterilizing substance S and the supply of the compressed air A to the nozzle 100 are carried out by two separate circuits.

With reference to Fig. 1 and Fig. 2, the nozzle 100 is adapted to generate a jet 110 comprising sanitizing and sterilizing substance S and compressed air A.

For example, the jet 110 may include a flow rate of compressed air A of 70-100 Nl/min (where normal conditions are defined at temperature T=0°C and P=1 atm) and a flow rate of sanitizing and sterilizing substance S of 200-500 ml/min. In particular, the nozzle 100 is adapted to nebulise or atomise the entering stream of sanitizing and sterilizing substance S by mixing it with an entering stream of compressed air A, whereby nebulisation or atomisation generally indicates the reduction of a liquid into very small parts, i.e. droplets.

The mixture of sanitising and sterilising substance S and compressed air A is passed through at least one outlet of the nozzle 100, typically through an orifice at the end of the nozzle 100. The passage of the mixture through the orifice of nozzle 100 generates a jet 110.

According to a preferred embodiment, the nozzle 100 is an ultrasonic nozzle.

Advantageously, the nozzle 100 further comprises an ultrasound generator, in particular one or more ultrasound generating blades, adapted to promote the creation of droplets of a sanitising and sterilising substance S within the jet 110. In particular, the droplets of sanitizing and sterilizing substance S are less than 10 micrometres in size.

Preferably, the droplets are smaller than 3 micrometres; a jet with droplets of substance smaller than 3 micrometres is typically called "dry fog".

More preferably, the droplets are smaller than 1 micrometre.

According to a preferred composition, the sanitizing and sterilizing substance S comprises hydrogen peroxide, at least one organic acid, at least one solvent, at least one surfactant and water.

In particular, said organic acid is preferably chosen from citric acid, acetic acid or a mixture thereof.

In particular, said solvent is ethyl acetate or an alcohol or a mixture thereof.

More specifically, said alcohol is preferably chosen from butanol or any isomer thereof, or ethanol.

The term butanol (or butyl alcohol) refers to an alcohol whose molecule contains four carbon atoms and whose brute formula is C4H10O.

There are four different butanol isomers:
- 1-butanol or n-butyl (normal-butyl) alcohol
- 2-butanol or sec-butyl (secondary-butyl) alcohol
- 2-methyl-1-propanol or isobutyl alcohol
- 2-methyl-2-propanol or t-butyl (tertiary-butyl) alcohol

For example, said solvent may be a mixture of ethyl acetate and 2-methyl-2-propanol (butanol isomer).

According to a preferred embodiment, said solvent is (2-methoxymethylethoxy)-propanol.

In particular, the surfactant is preferably an alkylpolyglucoside more preferably selected from branched C8 alkylpolyglucoside (AS 48), C11 alkylpolynucleoside (SL 11) or a mixture thereof.

According to a preferred formulation, the sanitizing and sterilizing substance S comprises the composition according to (EC) Regulation No. 1272/2008 reported in the table (advantageously dilutable in water):

| COMPONENT | CONTENT (w/w) |
|---|---|
| Hydrogen peroxide | > 3 % - < 5% |
| Citric acid monohydrate | > 1 % - < 5% |
| Sodium citrate | < 0.7% |
| Acetic acid | < 0.3% |
| (2-Methoxymethylethoxy)-propanol | > 1 % - < 3% |
| Ethyl acetate | > 0.1 % - < 1 % |
| Etidronic acid | < 0.3% |
| Alkylpolyglucoside (C8-C11) | > 0.5 % - < 3% |

It should be noted that formulations which differ by +/- 30% by weight, relative to the total weight of the formulation, from the values reported in the table may be considered as advantageously usable formulations.

Advantageously, the sanitizing and sterilizing substance S can be diluted in water.

Advantageously, the sanitizing and sterilizing substance S contains one or more additional components, such as water or perfume.

An example of a preferred formulation can be partially found in the Patent for industrial invention No. 102018000004475 entitled "Composizione detergente" in which the percentage of water in the aqueous composition (C) as described and claimed in the aforementioned Italian Patent can be replaced totally, or supplemented partially, with a percentage of hydrogen peroxide.

Advantageously, the sanitizing and sterilizing substance S is ejected from the nozzle according to predefined time cycles, such as once-twice a day; alternatively, the sanitizing and sterilizing substance S is nebulised or atomized as required, e.g. when a high percentage of contaminant is detected in the environment or when one or more people enter/exit the environment.

As shown in the figures, the tank (200 in Fig. 1 and 2200 in Fig. 2) is adapted to contain the sanitizing and sterilizing substance and to supply the nozzle 100 of the equipment through the duct 210.

If the tank of the equipment is interchangeable, it can advantageously be provided with an electronic tag (e.g. an "NFC tag") adapted to be read and preferably also written. In this case, wherein the electronic unit (referred to as 600 in Fig. 2) of the equipment is provided with means adapted to read and preferably also write the electronic tag.

The electronic tag can store:
- unique identification information of the tank, and
- information related to the content of the tank, in particular to the substance initially contained in the tank and preferably to the amount of substance initially contained in the tank.

The identification information (which is neither erasable nor modifiable) is preferably encrypted in such a way that the electronic unit can determine whether the tank (with its content) is original by decrypting it.

Advantageously, the electronic tag is adapted to be written permanently, i.e. information (in particular information about the tank content) can be added to the tag, but information cannot be removed nor changed. In this case, the electronic unit can be used to write the electronic tag by storing information about the amount of substance contained in the tank during the operation of the equipment. For example, a special status information can be stored on the tag only when the tank is empty (in particular, free surface below the minimum level), or status information of the tank filling level can be stored repeatedly on the tag (e.g. first 100% or maximum level, then 90%, ..., then 20%, and finally 10% i.e. minimum level). In this way, the tank can no longer be (abusively) reused nor (abusively) topped up with a substance, and therefore it is possible to ensure that sanitisation and/or sterilisation is only carried out by the equipment with the established substance and only according to the established protocol.

Typically, the equipment according to the present invention is adapted to specifically track and store the treatments carried out in particular through its electronic unit (referred to as 600 in the example of Fig. 2). In technical computer jargon, the term "log" (the sequential and chronological record of operations carried out and/or events occurred) may be used and refers primarily to treatments carried out by the equipment, but may also include any anomalies that have occurred in the equipment (e.g. indicated as "errors" by the control software of the electronic unit) and/or status conditions that have occurred in the equipment (e.g. liquid level in the tank and/or accumulator charge and/or connection to a telephone or computer network). With regard to the treatments, it may be useful to record: date and time of start, date and time of end, duration (which may also derive from the two previous pieces of information), substance used for the treatment.

It can be provided that the treatments and/or the treatment plan to be carried out by the equipment can be programmed not only locally, but also remotely.

The "log" will typically be stored, preferably in a secure and unmodifiable manner (possibly with the addition of a "digital signature" affixed by the equipment on behalf of the operator of the equipment), within the equipment, for example on a hard disk or in a removable memory (CD-ROM, memory card, ...). Furthermore, it can be provided that the "log" may also be stored outside the equipment, in memory media of another equipment which may belong for example to the owner of the treatment equipment or to the operator of the equipment or to a third entity which may be in charge of verifying the treatments carried out.

In the event of external storage and/or processing, the log may be transmitted outside the treatment equipment in various ways, such as by telephone connection (in particular by sending SMS or MMS messages) and/or computer connection (in particular WiFi connection to a computer network or direct 4G or 5G connection to the Internet or connection to the Internet via WiFi connection). The "log" can also be transmitted in small portions, e.g. as something new needs to be stored.

Storage and/or transmission may take place, for example, in an encrypted form. One or more of the functionalities described above will typically be implemented thanks to the equipment electronic unit, in particular thanks to its HW and/or SW.

## Claims

1. Equipment (1000, 2000) for nebulising or atomising a sanitizing and sterilizing substance (S) in an environment, said substance (S) being typically in liquid state, comprising at least one nozzle (100) having at least one inlet and at least one outlet and being adapted to eject said sanitizing and sterilizing substance (S);
wherein said nozzle (100) is fluidly connected to at least one duct (210) adapted to be supplied with said sanitizing and sterilizing substance (S), preferably under pressure, and is adapted to generate a jet (110) comprising said sanitizing and sterilizing substance (S);
wherein said sanitizing and sterilizing substance (S) comprises hydrogen peroxide, at least one organic acid, at least one solvent, at least one surfactant and water.

2. Equipment (1000, 2000) according to claim 1, wherein said organic acid is preferably selected from citric acid, acetic acid or a mixture thereof.

3. Equipment (1000, 2000) according to claim 1 or 2, wherein said solvent is ethyl acetate or an alcohol or a mixture thereof.

4. Equipment (1000, 2000) according to any one of claims 1 to 3, wherein said alcohol is preferably selected from butanol or an isomer thereof, or ethanol.

5. Equipment (1000, 2000) according to any one of claims 1 to 4, wherein said surfactant is an alkylpolyglucoside, preferably selected from branched C8 alkylpolyglucoside, C11 alkylpolyglucoside or a mixture thereof.

6. Equipment (1000) according to any one of the preceding claims 1 to 5, comprising:
- a plurality of nozzles (100) having at least one inlet and at least one outlet and being adapted to eject said sanitizing and sterilizing substance (S), and
- a hydraulic network adapted to distribute said sanitizing and sterilizing substance (S) preferably under pressure to said plurality of nozzles (100).

7. Equipment (1000, 2000) according to any one of the preceding claims 1 to 5, comprising at least one tank (200) adapted to contain said sanitizing and sterilizing substance (S) and to supply said nozzle (100) through said duct (210).

8. Equipment (1000, 2000) according to claim 7, wherein the tank (200) of the equipment is interchangeable and is provided with an electronic tag adapted to be read and preferably written, wherein the equipment comprises an electronic unit (600) provided with means adapted to read and preferably write said electronic tag, wherein said electronic tag stores:
unique tank identification information, preferably encrypted, and
information related to the content of the tank, in particular to the substance initially contained in the tank and preferably to the amount of substance initially contained in the tank.

9. Equipment (1000, 2000) according to claim 8, wherein said electronic tag is adapted to be permanently written, and wherein said electronic unit (600) is adapted to write said electronic tag by storing information related to the amount of substance contained in the tank, in particular an empty-tank state, during the equipment operation.

10. Equipment (1000) according to claim 7 or 8 or 9, wherein said tank (200) is adapted to contain said sanitizing and sterilizing substance (S) under pressure.

11. Equipment (1000, 2000) according to claim 10, comprising further at least one compressor (300) fluidically connected at inlet to said environment and at outlet to said tank (200).

12. Equipment (1000, 2000) according to any one of the preceding claims, further comprising at least one compressor (300) fluidically connected at inlet to said environment and at outlet to said at least one nozzle (100).

13. Equipment (1000, 2000) according to any one of the preceding claims, wherein said nozzle (100) is adapted to generate a jet (110) further comprising air (A).

14. Equipment (1000, 2000) according to any one of the preceding claims, wherein said nozzle (100) is adapted to disperse said sanitizing and sterilizing substance (S) in the form of droplets of a size of less than 10 micrometres, preferably less than 3 micrometres, more preferably less than 1 micrometre.

15. Equipment (1000, 2000) according to any one of the preceding claims, wherein said nozzle (100) further comprises an ultrasound generator adapted to promote the creation of droplets of said sanitizing and sterilizing substance (S) in said jet (110).
